# EUROPEAN PATENT APPLICATION

(11) **EP 1 739 423 A2**
(43) Date of publication of application: **03.01.2007**
(21) Application number: 06253446.6
(22) Date of filing: 30.06.2006
(51) Int. Cl.: G01N 33/38, G01N 29/44

(54) **A monitoring device**

(30) Priority: 01.07.2005 GB 0513591
(71) Applicant: Andrews, David Richard, Cambridge CB4 5NX (GB)
(72) Inventor: Andrews, David Richard, Cambridge CB4 5NX (GB)

(57) **Abstract**

A monitoring system uses sound waves and/or ultrasound waves that are emitted into a structure being monitored from one or more monitoring devices and echo-waves from the interior and surface of the structure are received at one or more monitoring devices at locations of importance to the stability and operational worthiness of the structure. Received signals from echo-waves are processed so that operational noise is largely eliminated or reduced to an acceptable level then collected and stored or archived; newly received and processed signals are also compared with existing archive signals and a decision is made for each new signal if it is representative of the structure being unchanged or representative of the structure after significant structural change has occurred. Results of decisions at locations of interest are combined to give a graded risk for the structure as a whole as well as identifying specific locations of significant structural change. An operator is warned when a threshold risk is reached allowing response actions to be initiated, for example: safety procedures and/or repair procedures.

## Description

The present invention relates to a system for monitoring the mechanical integrity and operational worthiness of structures, particularly large concrete structures such as bridges, power stations and dams but not necessarily limited to structures made of concrete and not necessarily large structures.

Large structures are expensive to build and they are generally designed to last between twenty years and one hundred years. Many structures of this kind have been built since 1950 and owners and operators of these structures have a strong financial incentive to prolong using them and to avoid demolishing them when they reach the end of their design-life. There is also a significant environmental cost associated with demolishing and rebuilding which it is desirable to avoid. Consequently, it is desirable to prolong using structures of this kind for as long as possible.

It is also desirable to use structures with reduced material quantities because this brings down the cost of construction, however, reduced material quantities can result in lower margins of safety.

When using a structure beyond its design life or with lower safety margins it becomes particularly important to inspect and to monitor its mechanical integrity and to review its operational worthiness more closely in order to maintain safe operating conditions. It is also financially advantageous to direct maintenance and repair to locations with the greatest need.

Clearly the quality of inspection and the frequency of inspection are important when reviewing the operational worthiness of ageing structures and lightweight structures. Current inspection procedures typically involve a visual examination, performed once every several years by an experienced structural engineer. Many experts consider this to be inadequate.

It would be desirable to inspect or monitor the mechanical integrity and operational worthiness of a structure by making use of information derived from the interior of the structure rather than its surface, since it is the interior that carries the load due to the weight of the structure and any operational loading. It would be desirable to inspect or monitor the mechanical integrity and operational worthiness of a structure at many locations and more frequently than once every few years; a more desirable frequency might be a few times a day but ideally the frequency should be chosen to suit the structure and its operating conditions.

It would also be desirable to use monitoring results in algorithmic criteria to judge or assess the mechanical integrity and operational worthiness of each particular structure, as developed by engineering experts for each structure. It would be desirable but not essential that the monitoring method could perform the judgement in the form of a risk assessment and to provide means for identifying locations on the structure where significant structural change had occurred and, consequently, where repairs would probably be more efficacious and cost-effective. It would also be desirable if the monitoring method kept archived records relating to the mechanical condition of the structure to assist in verifying to third parties, for example health and safety regulation inspectors or lawyers considering litigation relating to safety, whether or not the structure has remained in a stable mechanical condition over a certain period of time covered by the archive. Finally, it is important that the annual cost of running the monitoring method is low and that it is cost-effective when compared with current monitoring practice.

### STATE OF THE ART

Current inspection practice of structures generally involves infrequent visits by an expert, for example a structural engineer, perhaps with an interval of several years between visits. Unfortunately, several years can be long enough for an ageing structure to deteriorate significantly under adverse conditions. Visual inspection, commonly the basis of an inspection, is considered by many experts to be unreliable, since the internal condition of the structure remains substantially unknown and it is the interior of the structure, not the surface, that must bear the load of the structure and any operational loading. Certain classes of deterioration of structures are invisible from the surface of a structure, for example the corrosion of steel tendons in post-tensioned, reinforced concrete structures and tendons are the main load bearing components in this class of structure so current methods of inspection are thought not to provide reliable information for owners and operators of structures. There have been examples of notable failures of inspection, sometimes resulting in unexpected collapse of old and new structures, damage to property and loss of life.

The monitoring system described herein provides means for detecting a wide range of structural defects in the interior of the structure at any range from a test location from the surface to about 0.5 m to 2 m into concrete; monitoring can be concentrated at any number of locations identified by experts as important for assessing the mechanical integrity and operational worthiness of the structure and the frequency of testing at any given location can be chosen to be as high as several times an hour or lower than once a week.

It is known to inspect or monitor concrete structures by a number of other methods, including: thermography, gamma ray, X-radiography, vibration holography, neutron radiography, dye penetration, magnetic induction, electric potential mapping, radar or microwave probing, acoustic emission, ultrasonic time of flight, ultrasonic resonance spectroscopy, ultrasonic pulse-echo, ultrasonic guided-wave, and electromagnetic guided-wave testing. Many of these methods have disadvantages; for example, radar, or microwaves, cannot penetrate the metal duct surrounding post-tensioned tendons. X-rays can penetrate the tendon duct and detailed images of steel forms in concrete can be made but exposure times for creating an image are typically several hours and it may not be possible to keep the structure fully operational during exposure because of the radiation hazard inherent with X-rays.

Sound waves and ultrasonic waves are inherently sensitive methods for probing virtually all structural materials, including concrete. Because they are mechanical waves, sound waves and ultrasonic waves are intrinsically sensitive to the mechanical condition of any material; by way of contrast, electromagnetic waves are only sensitive to changes in the electrical properties of materials and are not, therefore, intrinsically suited to assessing mechanical properties. Sound waves and ultrasonic waves are also intrinsically safe: they are not ionising radiation, unlike gamma-rays, X-rays and microwaves.

The monitoring system described herein provides means for monitoring structures using sound waves, with sensitivity to small flaws or large flaws or emerging deterioration and can be run safely at all times during normal operation of the structure.

Ultrasound waves are known to be used for testing steel and most other metals. Recent advances in pulse-echo inspection of concrete have proved that sound waves and ultrasound waves are capable of probing concrete to depths about 1 metre, of finding reinforcement bars, tendon ducts, honey-combing, large cracks, networks of micro-cracks and structure walls. A sound wave and certain ultrasound waves take roughly 1 millisecond to travel out a distance of 1 metre and to return back to the point of transmission. So, by recording electrical signals derived from sound waves and ultrasound waves for periods of approximately 1 millisecond, or longer, using a transmitter and receiver located not more than 1 metre apart, information from a range of approximately 1 metre can be collected, which represents information from a mass of approximately 3 tons, if the structure is made of concrete. The range of penetration of sound waves and ultrasound waves is known to be restricted by both the degree of heterogeneity or graininess of the material composing the structure and the ratio of a size representative of the grains to the wavelength of the sound wave or ultrasound wave; the smaller the ratio of a grain-size to wavelength the greater the range. Therefore, the penetration range depends upon the wavelength or frequency of sound waves or ultrasound waves emitted into the material. Generally speaking, the longer the wavelength the greater the penetration. The degree to which a sound wave can detect a small flaw or feature also depends upon the wavelength, with smaller flaws requiring shorter wavelengths, hence higher frequencies. Clearly, when choosing the wavelength of sound or ultrasound to use in a test, there is a balance to be made between probing range and size of flaw to be detected but, for example, it is known that sound waves with frequencies in the range 10 kHz to 500 kHz can be used in various concrete types but higher frequencies provide better performance with metals.

A variety of sound or ultrasound pulse-shapes can be used for testing a given structure at any given location. It is known to use: as short a packet of waves as possible, determined by the impulse response of the transmitter; long (semi-infinitely long) bursts of sine-waves of virtually constant frequency and amplitude; intermediate length bursts of sine-waves of varying frequency and amplitude, sometimes known as chirps.

It is known in the field of inspection using sound or ultrasound that interpretation of the signals from a receiver follows after some stages of signal processing. Common stages of processing used commercially include: amplification, rectification, setting a fixed threshold for detection and display with final analysis by an operator. More recently computers have been used to perform other kinds of signal processing, for example: averaging, conversion of signals into the frequency domain (instead of the time domain), and image generation. Owners and operators of structures want monitoring devices with a high level of interpretation and decision making built into the monitoring devices.

The monitoring system described herein provides means for monitoring structures using computers to perform all the processing and much of the interpretation normally performed by an operator so that the monitoring system runs automatically without the need for an operator. This contributes substantially to reducing the annual or recurring operating costs of the system.

It is also known in the field of inspection to use ultrasonic transducers and to move or scan them over the surface of a structure under test in order to locate features of interest in the depth of the structure, such as cracks. It is also known to fix them for some period of time to monitor one position. An ultrasound transducer must be connected by its own cable to equipment necessarily used in testing, it is not normally possible to share cables between transducers without disconnecting all but one of the transducers; on a large structure it would not be possible manually to disconnect all but one transducer at a time and so share one cable. If cables were shared then signals from different transducers would interfere and render the results of no use. The requirement for each transducer to have its own cable returning to the test equipment makes it expensive to monitor a structure with many transducers because of the many cables that would need to be installed; if the structure were large, such as a bridge over a major river, requiring very many transducers to monitor it, perhaps one hundred or more, then the cost of installing and fixing the cables could easily be prohibitive and has generally made it impractical to use sound or ultrasound methods for monitoring structures. In addition, many sound or ultrasound transducers are only capable of driving electrical signals over relatively short distances of about a few metres so it is technically impossible to use transducers on a structure more than a few metres in size.

The monitoring system described herein provides means for substantially minimizing the total cable length or of eliminating cables entirely by providing means for communicating with many monitoring devices on one cable or means for communicating with many monitoring devices on a single, narrow-band radio channel. Therefore the monitoring system described herein can be installed at very much lower cost than a system requiring each transducer to have its own cable.

The method of acoustic emission has been used for monitoring structures using many receivers connected with their own cables back to a single equipment for analysing the signals from receivers; in the case of acoustic emission monitoring of offshore oil production structures the performance has been disappointing in at least one instance. Acoustic emission monitoring is different from the monitoring system described herein in two important ways: (a) acoustic emission detects the occurrence of short duration events, notionally when short pulses of sound are emitted by cracks as they advance through the structure; whereas the monitoring system described herein assess the mechanical effect on the structure resulting from the existence and advancement of the crack, the monitoring system herein detects material properties more directly relevant to the residual strength of the structure; (b) acoustic emission devices are sensitive to operational noise coming from the structure, for example the noise of waves striking offshore structures, and wave-noise is known to generate false alarms in acoustic emission monitoring devices; whereas the monitoring system described herein uses averaging of recorded signals and other signal processing strategies to eliminate substantially the effect of random, operating noise. Other kinds of structures have their own operational noise, for example, road bridges, rail tunnels and power stations so the problems experienced by acoustic emission on offshore structures is unlikely to be unique.

### STATEMENT OF INVENTION

Accordingly, in a system for monitoring the mechanical integrity, operational worthiness and safety of a structure there is provided: (a) at least one and preferably a plurality of monitoring devices, each with means for being attached and/or coupling to the structure to be monitored, so that a known pattern of sound waves and/or ultrasound waves can pass from one or more electro-mechanical transmitter transducers in one or possibly more monitoring devices into the structure being monitored and so that echo-waves of sound and/or ultrasound from the surface and interior of the structure being monitored can pass into one or possibly more monitoring devices to be converted there by one or possibly more electro-mechanical receiver transducers into electrical signals and then, preferably but not essentially, converted into digital signals, preferably but not essentially, signals processed to enhance the quality of the information relating to echo-waves therein, with, preferably but not essentially, means to store at least one and possibly more echo-wave signals in each monitoring device; (b) means for communicating information, including information from echo-wave signals, from one or more and preferably all the monitoring devices in a monitoring system to possibly one or more communicating devices or to possibly one or more of the monitoring devices but ultimately to preferably one but possibly more than one device to store or archive information derived from echo-wave signals from one or a plurality or preferably all the monitoring devices capable of collecting echo-wave signals on the structure to be monitored; (c) means in a monitoring system for analysing or interpreting some or all of the information pertaining to echo-waves collected by one or more and preferably all the inspection devices monitoring the structure to be monitored and preferably but not necessarily to compare the information, possibly but not essentially after some processing, with other processed or archived information in order to decide if any significant change in the mechanical integrity, operational worthiness and safety of the structure being monitored has happened; (e) means to alert an operator or a person or persons or a system about any changes in mechanical integrity, operational worthiness and safety of the structure being monitored; (f) means for performing some or preferably all the tasks of a monitoring system partially or preferably fully automatically; (g) means for providing power to the various monitoring devices, communicating devices and archive devices requiring power to function properly.

### CONSISTORY CLAUSES

A monitoring system has monitoring devices placed at locations that are preferably but not essentially chosen by a structural engineer or a person with expertise related to what the monitoring system is intended to monitor.

In some embodiments of monitoring systems, it is desirable but not essential, for reasons of reducing the cost, to have one or a plurality of monitoring devices, part of a monitoring system, that contain electro-mechanical transducers both for transmitting and for receiving sound or ultrasound waves in a single unit. It is also possible for one electro-mechanical transducer in each monitoring device to be used both for transmitting and for receiving sound or ultrasound waves, which results in a further reduction in the cost of a monitoring device.

In some embodiments of monitoring systems it is desirable but not essential to attach one or a plurality of monitoring device to the surface of the structure being monitored for a period of time that is equal to or greater than the desired monitoring period, which might be as short as a few minutes but equally could be as long as many years. In this way the location of actuation of the sound and ultrasound transducers remains virtually constant, which makes it easier to decide if a significant change has occurred in the structure being monitored under any monitoring device.

In certain embodiments of a system for monitoring the mechanical integrity and operational worthiness of a structure, it is desirable but not essential to use one or possibly more demountable monitoring devices, which can be moved between test locations.

In certain embodiments of a system for monitoring the mechanical integrity and operational worthiness of a structure, there is preferably one but possibly more monitoring devices, one example being a demountable monitoring device, it is also desirable but not essential to fix permanently to the structure being monitored at least one but preferably a plurality of attachment devices to aid or simplify the mounting or demounting, of any inspection device. Each attachment device is, preferably but not essentially, a simple, low-cost device, for example a plate, fixed to the structure to mark or locate or otherwise define the precise location of transmitting and/or receiving sound waves or ultrasound waves, with the attachment preferably but not necessarily assisting in simplifying the coupling of sound waves or ultrasound waves into or out of the structure.

In certain embodiments of a system for monitoring the mechanical integrity and operational worthiness of a structure, there is one or preferably a plurality of monitoring devices in each of which it is desirable to include a mating surface part, preferably but not necessarily a plate, which is used next to the surface of the structure being monitored.

In certain embodiments of a system for monitoring the mechanical integrity and operational worthiness of a structure, it is desirable to fill any gap between the surface of a structure being monitored and any mating surfaces of any permanently fixed monitoring devices or the mating surfaces of any permanently fixed attachment devices to aid the efficient transmission of sound or ultrasound between the monitoring devices or the attachment devices and the structure being monitored. Mating surfaces are preferably but not necessarily made of a material to which the coupling agent adheres; it is desirable that the coupling agent also adheres to the material of which the structure, under the mating surfaces, is made. The coupling agent should preferably fill the space between the sound or ultrasound transmitters and/or receivers in the monitoring devices and the structure surface.

In certain embodiments of a system for monitoring the mechanical integrity and operational worthiness of a structure, it is also desirable but not essential that a coupling agent hardens to fix and hold the monitoring device or the mating surface of an attachment in one place on the surface of the structure under test for a relatively long period of time possibly for as long as the period of monitoring so that it supports the weight of the monitoring device and withstands any operational or other forces that might be exerted on the monitoring device.

In certain embodiments of a system for monitoring the mechanical integrity and operational worthiness of a structure, it is also desirable but not essential that any hardened coupling agent is acoustically matched to the material of which the structure being monitored is made under the mating surface of the monitoring device or attachment device.

In certain embodiments of a system for monitoring the mechanical integrity and operational worthiness of a structure, there is one or preferably a plurality of monitoring devices or attachment devices with which it is desirable but not essential to use two or possibly more different means to support the weight of each monitoring device or attachment device that is fixed to a structure being monitored, particularly when a location for a monitoring device or attachment device is chosen that presents a risk of causing injury or damage in the event that the monitoring device or attachment device should fall from the structure under the force of gravity or some other force, if one of the supporting means were to fail.

In certain embodiments of a system for monitoring the mechanical integrity and operational worthiness of a structure, there is one or a plurality of monitoring devices or attachment devices with which it is desirable but not essential that one of the different means of supporting the weight of each monitoring device or attachment device can quickly support the weight and any operational or other forces that might be exerted on each monitoring device or attachment device. This is particularly useful when the second means of fixing the monitoring device or attachment device is an adhesive that takes time to develop sufficient strength to support the weight of the monitoring device or attachment device because during that time the fixing means that quickly supports the load can support each monitoring device or attachment device alone.

In certain embodiments of a system for monitoring the mechanical integrity and operational worthiness of a structure, made in part or entirely of concrete, it is desirable but not essential for any parts of any monitoring devices, carrying or conveying sound waves or ultrasound waves, to be made in part or possibly entirely out of a composite material of a hardened mixture of epoxy resin or a similar thermo-setting or thermo-plastic polymer and alumina powder or some similar mineral powder, because the composite material can be acoustically matched to concrete and because mortars adhere to it.

In certain embodiments of a system for monitoring the mechanical integrity and operational worthiness of a structure, possibly but not necessarily made in part or entirely of concrete, it is desirable but not essential to use mortar or similar viscous liquid-like adhesives as coupling agents because mortar and similar viscous liquid-like adhesives adhere to concrete when liquid-like and when hardened. Mortar is also acoustically matched to concrete. Furthermore, by using viscous liquid-like adhesives, which flow, any gaps between mating plates and attachment devices and any uneven surface of the concrete at the test location can all be substantially or entirely filled as the mortar or similar adhesive flows but with sufficient viscosity in the adhesive to prevent significant loss of adhesive from between the mating surfaces.

In certain embodiments of a system for monitoring the mechanical integrity and operational worthiness of a structure, there is one or preferably a plurality of monitoring devices or attachment devices in which it is desirable but not essential to make any mating components thereof with one hole or possibly more, conveniently but not necessarily located in the centre of each mating component, so that some means of rapidly holding any monitoring device or attachment device onto the surface being monitored can use the hole or holes to support the weight of a monitoring device or attachment device.

In certain embodiments of a system for monitoring the mechanical integrity and operational worthiness of a structure, there is one or preferably a plurality of monitoring devices in which it is desirable but not essential to use anchor bolts or similar devices to hold mating plates or attachment devices in place on the surface of the structure being monitored.

In certain embodiments of a system for monitoring the mechanical integrity and operational worthiness of a structure, it is preferable but not essential that any monitoring device has an enclosure to protect the electronic circuits of the monitoring device.

In certain embodiments of a system for monitoring the mechanical integrity and operational worthiness of a structure, it is preferable but not essential that any monitoring device has an enclosure that is impervious to water, other chemicals, dust, the rays of the sun, wind and that it should be rugged to withstand a variety of environments, preferably but not essentially for many years.

In some embodiments of a monitoring system it is desirable to use a cable, preferably an electrical cable or possibly a fibre-optic cable, for conveying information and possibly power between one or more and preferably all monitoring devices in the monitoring system and possibly to other communicating devices and ultimately to one or possibly more archive devices and possibly to one or more sources of electrical power. It is further possible to share one or possibly more cables between two or a plurality or preferably all monitoring devices, archives and communicating devices in such a pattern as to reduce or substantially to minimize the total length of cable used in a monitoring system, with the benefit that the cost of installation is lower if the total cable length is small. One preferred arrangement of cable involves linking each monitoring device to a nearby neighbouring monitoring device, preferably but not essentially connecting other communicating devices in the same way and preferably but not necessarily with at least one archive device connected in the same way; this arrangement of connections is sometimes known as a daisy-chain. It is preferable to use a cable in this way because it can result in saving on cable length or time spent during installation compared with wiring each monitoring device separately to an archive device.

In some embodiments of a monitoring system it is desirable to use a cable, preferably an electrical cable or possibly a fibre-optic cable, with clusters of monitoring devices connected by cable to a communicating device, with the communicating device linked possibly to other communicating devices but ultimately to at least one or possibly more archive devices and possibly to one or more sources of electrical power. A plurality of cabling arrangements are possible, all of which result in saving on cable length or time spent during installation compared with wiring each monitoring device separately to an archive device.

In some embodiments of a monitoring system it is desirable to use radio as a communication means because radio eliminates the need for a cable for communications purposes, which substantially reduces the cost of installation.

In some embodiments of a monitoring system it is preferable but not essential to use substantially the same message-handling protocol for communicating between monitoring devices, communication devices and archive devices, which protocol, preferably but not essentially, seeks to avoid collisions between messages and is, preferably but not essentially, able to recover from messages corrupted by collisions between messages from different devices or other causes of interference.

In some embodiments of a monitoring system it is desirable but not essential to use one or a plurality of internal batteries in one or preferably a plurality of monitoring devices or communication devices, particularly but not essentially, when the monitoring device or any communication device does not have a cable to provide power.

In some embodiments of a monitoring system it is desirable but not essential to use one or a plurality of internal re-chargeable batteries in one or preferably a plurality of monitoring devices or communication devices and it is desirable but not essential to provide means for recharging any battery derived from power possibly from a cable but preferably in the environment, including but not limited to: means to convert power from the rays of the sun into electrical energy; means to convert power from the wind into electrical power; means to convert vibrational energy in the structure into electrical power, using possibly but not necessarily one or more piezoelectric or electro-magnetic devices; means to convert thermal gradients or temperature changes in the vicinity of the monitoring device into electrical power, using one or more Peltier effect or pyro-electric devices or a layered device, with dissimilar thermal expansion coefficients and at least one layer being a piezoelectric device.

In certain embodiments of a system for monitoring the mechanical integrity and operational worthiness of a structure, it is desirable to transmit one or a plurality of known patterns of sound waves or ultrasound waves into the structure being monitored, using electro-mechanical transducers in each of any monitoring devices with the capability to transmit. It is desirable but not essential to detect preferentially the same known patterns in any echo-waves from the structure being monitored, as collected by electro-mechanical receiver transducers in each of those monitoring devices with the capability to receive.

In certain embodiments of a system for monitoring the mechanical integrity and operational worthiness of a structure, a preferred pattern of sound waves or ultrasound waves to transmit from one or a plurality of inspection devices are semi-continuous sine-waves of constant amplitudes and frequencies because the presence of a continuous sine-wave can be found in any echo-wave using a narrow, band-pass filter having a centre frequency equal to the known transmitted frequency; in particular, preferably but not essentially, using digital frequency synthesis of the transmitted sine-wave and, preferably but not essentially, lock-in amplifier methods it is possible to create accurate, narrow band-pass filters capable of tracking any changes in the frequency of any transmitted sine-wave. It is preferable but not essential to measure the absolute amplitude of any received sine-wave signal and possibly the relative phase angle of any received sine-wave signal compared with the known transmitted wave. The use of a narrow acceptance range filter rejects signals at other frequencies, such as interference or operational noise in the structure being monitored, and thereby improves the reliability of assessment of structural integrity made by the monitoring system. It is also desirable but not essential to test at a plurality of frequencies from any given transmitting inspection device and to group the collected data, of frequency and possibly amplitude and possibly phase angle, for each frequency of the plurality tests, into one set of data for subsequent processing and archiving.

In certain embodiments of a system for monitoring the mechanical integrity and operational worthiness of a structure, a preferred class of pattern of sound waves or ultrasound waves to transmit from one or preferably a plurality of inspection devices is a burst of waves of finite duration, known herein as a chirp. One or more receiver transducers are synchronized to start collecting chirp echo-wave signals from transmitters in appropriate inspection devices with some controlled delays, preferably but not necessarily zero time delay after each chirp transmitter first starts transmitting, with chirps covering, preferably but not essentially, as wide a range of frequencies as possible but limited to the range of frequencies within which the transmitter and receiver transducers in the monitoring device or devices have useful sensitivity and further limited to the useful range of frequencies that can be transmitted through the material of which the structure being monitored is made. It is preferable to process received chirp echo-wave signals to extract chirps preferentially, which resulting signals having short, simple event pulses for the arrival of each chirp echo-wave, and the wider the bandwidth of the chirp the narrower, generally, will be the event pulse; narrow event pulses provide better resolution of echoes from reflectors in the structure being monitored which makes it easier to identify structural changes in the structure being monitored because structural changes generally cause changes in reflection of waves.

In certain embodiments of a system for monitoring the mechanical integrity and operational worthiness of a structure, particularly a structure made in part or entirely with concrete, a preferred pattern of sound waves or ultrasound waves to transmit from any inspection device is a chirp with a swept-frequency lasting preferably but not essentially more than one period or cycle of the waves transmitted, for example a time in the range 30 microseconds to 300 microseconds and covering a range of frequencies somewhere in the range from 10 kHz to 500 kHz. There is scope to adapt chirps to suit different concrete mixtures, for example, using lower frequencies with larger grain sizes in the material so that the wavelength of the chirp sound or ultrasound wave in the material is longer than the grain size; there is scope to adapt different chirps to suit other types of materials too.

In certain embodiments of a system for monitoring the mechanical integrity and operational worthiness of a structure, it is preferable but not essential to process echo-waves collected by receiver transducers in several stages when chirp patterns are being transmitted. The first two preferred stages of processing, after, possibly, signal amplification and filtering using analogue electronic circuits, are: averaging and pattern detection; these stages are preferably but not necessarily done in digital signal representation. Averaging has the effect of suppressing both random interference and operational noise compared with the echo-wave signals from the structure being monitored caused by systematically transmitting chirp patterns from an inspection device. Pattern recognition further suppresses interference and operational noise and compresses the duration of chirp signals into short, simple event pulses.

In certain embodiments of a system for monitoring the mechanical integrity and operational worthiness of a structure, a preferred method of pattern recognition that can be used with chirp echo-wave signals from inspection devices is sometimes called matched filtering; it is the optimum linear signal processing method for detecting a known burst of waves. Matched filtering can make use of a copy of the transmitted excitation pattern, however, it is preferable but not essential to make use of part of the signal collected during a calibrating test on a simple homogeneous sample, having the same or similar acoustic properties to the structural material of interest, because the original electrical chirp pattern is modified both by the transmitter as it is converted into a sonic or ultrasonic chirp wave pattern and by the receiver as the sonic or ultrasonic chirp wave pattern is collected and converted back into an electrical signal; the original excitation signal may not be an accurate copy of the original electrical drive pattern so an experimentally collected chirp is generally more accurate. The more accurately the matched filter is matched to the chirp pattern found in chirp signals from the structure under test then the simpler and sharper will be the resulting event markers after processing and the better will be the resolution of the internal components or reflectors of the structure being monitored and better resolution of structural details results in better sensitivity to structural changes.

In certain embodiments of a system for monitoring the mechanical integrity and operational worthiness of a structure, even if spike electrical excitation of the transmitters in one or a plurality of monitoring devices is used it is still preferable to use matched filtering based upon patterns recorded in one or a plurality of calibration experiments, as described earlier here. With spike excitation most if not all transmitter transducers generate sound or ultrasound waves that are substantially different in time representation to the electrical spike because a spike covers a wide range of frequencies but transmitters can only transmit waves in its, generally, much narrower bandwidth. The pulse of waves generated after spike excitation can still be efficiently detected by matched filtering.

In certain embodiments of a system for monitoring the mechanical integrity and operational worthiness of a structure, it is relatively unimportant in which order averaging and matched filtering are performed because they are both linear operations which commute with addition. The result of performing averaging first followed by matched filtering should be the same as matched filtering every signal then averaging. However, with other forms of pattern recognition the process may not be linear in which case it may be important in which order averaging and pattern recognition is done and possibly but not necessarily it will be important that averaging is done before pattern recognition.

By detecting known patterns of echo-waves generated in response to a transmitted signal a monitoring device of the kind described herein is better able than the acoustic emission method to work successfully in the presence of the normal operational noise of the structure being monitored because it uses the detection of a known pattern at precisely known times. Sound waves or ultrasound waves caused by operational noise and acoustic emission sources are very unlikely indeed to result in received signals that have the same pattern as that transmitted by inspection devices; pattern recognition rejects uncorrelated signals like operational noise and acoustic emission.

In addition, the monitoring system described herein is better able than acoustic emission methods to monitor the mechanical integrity and/or operational worthiness and/or safety of a structure because it is sensitive to the mechanical composition of the structure or the residual mechanical state of the structure, which is directly associated with its mechanical integrity; whereas acoustic emission can only indicate the number events of acoustic emission and it is not straightforward to relate this to the residual strength.

In certain embodiments of a system for monitoring the mechanical integrity and operational worthiness of a structure, it is preferable but not essential to follow averaging and pattern recognition by a stage of processing to convert bipolar signals derived from echo-waves from the structure being monitored into unipolar signals. A preferred method is to calculate the magnitude of the analytic function of the signal. Other possible methods include simple rectification or taking the magnitude of the signal, these latter two are preferably but not essentially followed by low-pass filtering or band-pass filtering to smooth the signal. It is desirable that unipolar conversion follows matched filtering.

In certain embodiments of a system for monitoring the mechanical integrity and operational worthiness of a structure, there is one or a plurality of monitoring devices in which it is preferable but not essential that there are data processing devices, preferably but not necessarily: a computer or a microprocessor or a digital signal processor or a microcontroller or combinations of the aforementioned devices along with suitable memory and other electrical circuits. The said data processing devices preferably but not necessarily control some or all of the following functions: the transmission of any sound and ultrasonic waves, the receiving of any sound or ultrasound waves, the conversion of any received waves into electrical signals, recording of received electrical signals, the processing of received electrical signals, communicating information ultimately to an archive device.

In certain embodiments of a monitoring system it is preferable but not essential that any archive device has one or possibly more data processing devices, preferably but not necessarily: a computer or a microprocessor or a digital signal processor or a microcontroller or combinations of the aforementioned devices. The said data processing device has means to perform substantially all of the following functions: communication with monitoring devices and communication devices, archiving of signals or information from monitoring devices, signal processing, decision-making about whether or not any significant structural change has occurred based upon information or signals from one or a plurality of monitoring devices and archive information or signals, combining of results of two or more decisions relating to different monitoring locations on the structure being monitored to create a decision for a larger part or larger parts of the structure being monitored or of the whole structure, communicating results of decision-making to an operator or to the public or to any system responsible for responding to such an event including but not limited to safety or repair of the structure.

In certain embodiments of a monitoring system, it is desirable to use the inspection devices to collect and archive information from the structure at a time when the structure is known to be free of significant structural faults, possibly but not necessarily when the monitoring system is first installed; independent tests may be needed to verify that the structure is free of structural faults. In certain other embodiments of the system the structure may be known to contain some limited faults and it is still desirable to use the inspection devices to collect and archive information from the structure. The data so archived can be used to help decide when a significant structural change has occurred.

In certain embodiments of a monitoring system there is means for decision-making, preferably but not necessarily, in an archive device using means known as an artificial neural network, with one or a plurality of artificial neural networks existing therein and each one specially trained to make decisions about one specific monitoring location of the plurality of monitoring locations. The basis of the decision-making is to compare the a newly received signal from a particular monitoring device, preferably but not necessarily processed to reduce interference, operational noise and to enhance the resolution sound or ultrasound echoes from structural components in the structure being monitored, with two or more sets of similar signals derived from the same monitoring location in the same signal format, with the two minimum sets being: (a) experimental signals, possibly processed, but retrieved from archive and collected before significant mechanical change and (b) some or preferably all the same experimental signals as in (a) but modified to be made to be representative of significant mechanical change, a process called migration herein. The decision-making algorithm decides which set the new signal is most like, preferably but not essentially, with the result of the decision given in the form of a probability that the signal under consideration belongs to the set characterized by significant structural change.

In certain embodiments of a monitoring system, it is desirable to take copies of archive information signal from monitoring devices taken when the structure is in a known condition, possibly but not necessarily a state free of faults or a state with known acceptable faults and to modify them, by a process is referred to as migration herein, so that migrated information signal becomes representative of the structure when in a mechanical state that is significantly changed. Migrated information and unaltered information can preferably but not necessarily be used for training decision-making algorithms used by the monitoring system. A variety of methods of migration are desirable but not essential, for example: (a) possibly add or possibly multiply a plurality of mean RMS levels of white-noise or multiplicative pink-noise above a chosen level to the information signal being migrated, this kind of migration has been found to be representative of concrete that has been damaged due to micro-cracking, and is sometimes associated with frost-damage or corrosion of steel forms in the concrete but other damage mechanisms in other kinds of structures also cause the same changes to information signals; (b) determine the positions of peaks in the information signal and cause the positions to be moved by a random amount above a threshold level, this has been found to be representative of concrete in which some change in geometry has occurred due to drilling, or spalling, or humidity changes but other damage mechanisms in other kinds of structures also cause the same changes to information signals.

In certain embodiments of a monitoring system, it is preferable but not essential, to use another method of migrating received signals, called finite element modelling (FEM) migration herein, that has the advantage that an expert, for example a structural engineer, can influence the type of damage that will be introduced by migration. FEM migration is effected as described below: (a) a FEM program is used to create a geometrically accurate, local model of the part of the structure being monitored in contact with a specific inspection device of interest, a dimensional accuracy of +/-2% can be achieved generally in the model, (b) the FEM program can then be run to predict the electrical signal to be received by the receiver transducer in the inspection device under consideration in response to the excitation applied to the transmitter signal, using the geometry model and the material properties of the various materials locally in the structure and preferably the material properties of transmitter and receiver transducers, (c) the material properties typically required by FEM are: density (+/-10%), Young's modulus (+/-25%), shear modulus (+/-50%) and acoustic or ultrasonic attenuation (+10000% to ― 99%); shown in parentheses are the typical errors for these values that might be known at the start of the modelling process, errors in material properties are larger than errors in geometry so it is reasonable to assume that errors in material properties cause a greater difference between any FEM migrated and unmigrated experimental signals, preferably but not necessarily, by means of a separate controlling program, it is possible to run the FEM program repeatedly with a fixed geometry but with material properties varied by the controlling program and chosen to vary within an error range specified by the expert or structural engineer so that a plurality of provisionally migrated signals can be created, (d) after a newly migrated signal is generated by the FEM program for a specific monitoring location it is desirable to compare it with the unmigrated experimental signal for that location and to calculate a scalar error value to quantify any differences, (e) the controlling program operates in such a way as to try to minimize this error, it is possible but not essential to use an algorithm called a genetic algorithm as the controlling program, The controlling program should keep varying the material properties in such a way as it judges should minimize the error until an acceptable error has been achieved, (f) at which point the provisionally migrated signal should appear to be substantially like the experimental signal and it is probable that the material properties used to make the last migrated signal are a more accurate representation of the material properties in the part of the structure under consideration, so the error in material properties has been reduced substantially, (g) the expert or structural engineer can then introduce one or possibly a plurality of different changes into the FEM model for the part of the structure under consideration, preferably but not necessarily to model faults or flaws or changes that would be of structural significance and which it would be desirable for the monitoring system to detect, and then to re-run the FEM program once for each of the faults or combination of faults resulting in one or preferably a plurality of new migrated information signals for that test location all representative of a significant structural change. Suitable control programs for stage (c) include: minimization algorithms, genetic algorithms, self-annealing minimizations algorithms.

In certain embodiments of a monitoring system, it is preferable but not essential, to have means to quantify the risk of significant structural change in a part of a structure monitored by an inspection device, preferably but not essentially, so that the risk is quantified as a probability of significant structural change. Consequently, it is preferable but not essential to use decision-making algorithms such as artificial neural networks or fuzzy logic which give decisions quantified as probabilities but these are not the only possible choices.

The risk to a structure is greater when significant structural changes happen in two or more monitoring locations, for example: significant changes in two different monitoring locations are of greater risk than significant change in one monitoring location, significant changes in three different monitoring location are of greater risk than significant changes in two monitoring locations, significant changes in certain monitoring locations may be a greater risk than significant changes in other monitoring locations, significant changes in two physically adjacent monitoring locations may be of greater risk than significant changes in two distant monitoring locations, significant changes that occur in two adjacent monitoring locations in a short period of time are of greater risk than changes that happen over a longer period of time and so on for all possible combinations of monitoring locations. A structural engineer has the skill and experience to best consider the consequences of significant change on the risk to the structure as a whole. Consequently, in certain embodiments of a monitoring system, it is preferable but not essential to have means to combine risks for two or more inspection devices to give a risk for the entire structure being monitored and for risks to be combined in a way that is controlled by a expert in the stability of the structure being monitored.

In certain embodiments of a monitoring system, it is preferable but not essential, for monitoring devices to have means for measuring the temperature of the surface of the structure and for this temperature value to be included with echo-wave information, ultimately to form a part of the information to be submitted to a decision-making algorithm both for training and for decision-making. The advantage of this approach is that seasonal and even daily temperature changes can alter the material properties of surface layers of structures, causing received information signals to be changed. Without taking temperature into account it is possible for a decision-making algorithm to mistakenly associate a change in temperature as a significant change in structural integrity but a well-trained decision-making algorithm compensates for the variation in temperature so that the frequency of these false alarms of significant change in structural integrity is reduced or eliminated.

In certain embodiments of a monitoring system, it is preferable but not essential, for a monitoring device to have means for signalling its presence amongst two or a plurality of monitoring devices for the purpose of identifying its location on the structure being monitored. The means for signalling is preferably optical but might also be audible or possibly electromagnetic or possibly other means. One preferred but not essential method of signalling is to use a lamp that is visible from the outside of the monitoring device that can be flashed at a speed under the control of a processor within each monitoring device. It is also possible but not essential for the signalling to be modulated to contain information, such as information that can allow an association with the identifier or name or address or some such similar of the monitoring device.

In certain embodiments of a monitoring system, it is preferable but not essential, that a monitoring device using cable communications may have different means for connecting to the cable. One possibility is to fit two electrical sockets into the housing of the monitoring device so that a cable can be simply plugged in; another possibility is to have a short length of cable emerging from each sensor and to join the short length to the cable travelling between other sensors. The former method is better suited to monitoring systems to be used in sheltered environments and where precise cable length is not critical; the latter method may be better suited to outdoor, hostile environments and where the cable length must be kept to a minimum.

In certain embodiments of a monitoring system, particularly monitoring systems for use on structures made partly or entirely of concrete, it is desirable to embed one or a plurality of inspection devices in the concrete of which the structure is made, preferably but not essentially, close to an outer surface of the structure through which communications means are possible, possibly in the form of communications using a cable or possibly using communications by radio or some other electro-magnetic wave, with means to provide power to each inspection device possibly through a cable or possibly derived from environmental sources.

### SPECIFIC DESCRIPTIONS

Six specific embodiments, A, B, C, D, E and F of the invention will now be described with reference to the accompanying drawings.

In embodiment A, in which:
Figure 1 shows a schematic diagram of a monitoring system using cable communications, comprising several monitoring devices fixed to a structure, interconnected by a single daisy-chain cable with a single archive device at one end of the cable.
Figure 2 shows a sketch of the principal components in a monitoring device for use on a structure.

Referring to drawing 1 the monitoring system comprises a number of monitoring devices, all marked 2, attached to a structure, 1, with an archive device 3 contained in a building or shelter, 4, a distance away from the structure. Each monitoring device is capable of both transmitting and receiving sound waves. Information and power can be communicated over a cable, 5.

Referring to drawing 2, there is a monitoring device which is mechanically attached to the structure, 1, using a combination of an anchor bolt, 6, fixed into a pre-drilled hole in the surface of the structure and holding a mating plate, 7, which is fixed into the monitoring device enclosure, 8. The mating plate, 7, enclosure, 8, and top plate, 14, protect the internal parts of the monitoring device against penetration of water, dust and other environmental agents whose presence on the electronic circuits, 13, might cause them to perform not as intended. Access to tighten the anchor bolt through the monitoring device is provided by a tube, 15, sealed against water and dust with the mating plate, 7, and top plate, 14. The monitoring device is further held in place by additional means, a layer of hardened adhesive, 9, between the mating plate, 7, and the surface of the structure, which in the case of a structure, 1, with a concrete surface can be mortar-like adhesive. The adhesive, 9, is applied in a form that is substantially of a viscous liquid so that it flows to fill most if not all of the surface relief of the structure, 1, and the gap between the mating plate, 7, and the surface of the structure, 1, but without flowing substantially away while bonding to the surface of the structure, 1, and also to the mating plate, 7, which plate and transducers, 10 and 11, in the case of a structure, 1, with a concrete surface, are preferably made in part or entirely with a composite material of epoxy-resin and alumina filler that is acoustically matched to concrete and to which mortar bonds. The weight of the monitoring device is supported temporarily by the anchor bolt, 6, during the hardening of the adhesive, 9, and thereafter by both the anchor bolt, 6, and the adhesive, 9. It is important that the hardened adhesive, 9, covers the sound or ultrasound transducers, 10 and 11, because it is a coupling agent to allow ultrasound to pass easily between the transducers, 10 and 11, and structure, 1. The monitoring device has two transducers, 10 and 11, acting as transmitter and receiver respectively. The transmitter and receiver, 10 and 11, are fixed and sealed into the mating plate, 7. A small temperature sensor, 12, is fixed in the mating plate, 7, close to the surface with the adhesive,9, and close to the expanding bolt, 6. Electronic circuits, 13, are held inside the enclosure, 8. A cable, 5, brings power and communication signals. The electronic circuits, 13, have a digital signal processor device and memory, waveform synthesis devices, amplifiers, analogue-to-digital converter, cable communications devices and power management devices. The cable, 5, emerges from a top plate, 14, of the housing in which it is sealed against environmental ingress. The top plate also has a similarly sealed lamp, 22, fixed in it, which is used to signal for identification purposes.

In use one or more and generally many monitoring devices, 2, are attached to different locations on a structure, 1, during a process of installation. Each monitoring device, 2, is installed at a location chosen by an expert to be useful for the ultimate monitoring purpose. Each monitoring device, 2, is given an unique identifier or address or name, differentiating it from all the other monitoring devices, 2, so that it can communicate without ambiguity with other monitoring devices, 2. The location of each monitoring device, 2, is recorded with reference to a mechanical drawing of the structure, 1, cross-referencing the logical identifier with the physical position by means of an optical signal from the lamp, 22, in each monitoring device, 2, in turn; as well as having a paper copy of the mechanical drawing there is also a computer representation of the structure with representations of each monitoring device, 2, held on the archive, 3. Since a monitoring test, using sound or ultrasound waves, can be performed either between two monitoring devices, 2, or by one monitoring device, 2, on its own, with each testing different volumes of the structure, 1, it follows that it is possible for there to be more monitoring volumes than monitoring device, 2, locations. Once all the monitoring devices, 2, are attached to the structure, 1, to be monitored, the archive device, 3, is configured by an operator so that it is has information about the location, capabilities and address of each monitoring device, 2, and a schedule of tests for each monitoring device, 2. The monitoring system is then run on a commissioning basis, for some time, preferably but not necessarily for a few months during which time the monitoring system is run not only to test its reliability and proper functioning but also to archive some initial received signals. As part of the installation it is desirable but not essential to perform a thorough inspection of the structure so that any defects are identified and preferably, but not necessarily, repaired. It is important that the structure is in a known condition at the time of installation and, generally, the structure is in good condition; for the purpose of the remainder of this description the condition of the structure will be assumed to be good but this assumption is not a necessary condition for using the monitoring system. Copies of the initial data are migrated so that they are no longer representative of the structure in good condition but become representative of the structure after significant structural change. Artificial neural networks are then created on the archive, 3, and trained for every test volume for which there is initial data and migrated data. During the commissioning stage of the monitoring system a risk combination is decided for the structure, which is created and configured with the assistance of an expert structural engineer. The procedure involves the expert assessing the risk to the structure for each and every particular test volume exhibiting significant structural change, as detected by its appropriate artificial neural network, and each and every combination of risks from two or more test volumes; some test volumes may be more important than others and so will have a greater risk rating or weight. The expert also decides upon a critical risk rating level for the whole structure; if this level of risk is reached then the monitoring system issues one or a plurality of warnings in different forms, including, possibly: an audible alarm, an electronic message, a visible alarm, or any other automatic action, to an operator for safety, repair or any other purpose. The display of information, possibly but not necessarily, on the archive, 3, includes the aforementioned computer representation of the structure on a computer screen with representations of some or preferably all the inspection devices, 2, with colour coding of inspection devices, 2, and test volumes to show where significant changes have occurred. This marks the end of preliminary commissioning of the monitoring system and at this point the artificial neural networks and the risk analysis system are started so that an initial risk rating is determined for the structure. The expert follows the operation of the monitoring system over a preliminary period thereafter to check that the monitoring system is working correctly. It is also desirable but not essential that as newly received signals are archived they are also used for improving the training of the artificial neural networks, including migrated versions of signals, since this should improve the quality of the decision-making and make a system that is more robust against temperature changes over long period of time, such as daily and seasonal periods of time. By training the artificial neural networks with signals from the commissioning period only it is possible to detect significant changes that happen or emerge slowly; by continuously re-training the artificial neural networks with recent data that, preferably but not essentially, has not resulted in a significant change then it is possible substantially to ignore slow changes and only detect changes that happen quickly. Consequently, there is a range of ways of training the decision-making algorithms to detect changes with particular characteristics related to the seasons, time of day, loading patterns, so there is flexibility in how the system is used.

### In embodiment B, in which:

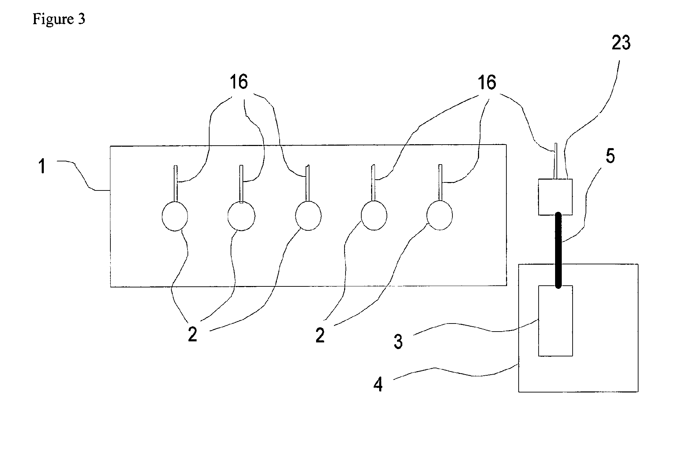

Figure 3 shows a schematic diagram of a monitoring system using radio communications, comprising several monitoring devices fixed to a structure, each monitoring device has its own radio antenna, with a communicating device able to communicate both by radio and by cable and the cable connected to a single archive device.

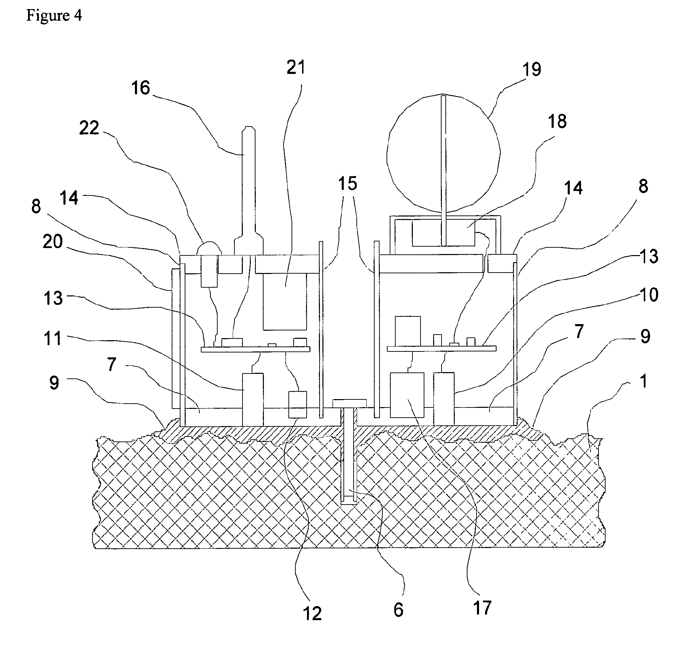

Figure 4 shows a sketch of the principal components in a monitoring device using radio communications.

Referring to drawing 3 the monitoring system comprises a number of monitoring devices, all marked 2, attached to a structure, 1, with the monitoring devices, 2, communicating with a communicating device, 23, and via a cable, 5, to an archive device, 3, contained in a building 4 a short distance away from the structure. Each monitoring device is capable of both transmitting and receiving sound and ultrasound waves. Information and recordings can be communicated by radio with antennas marked 16.

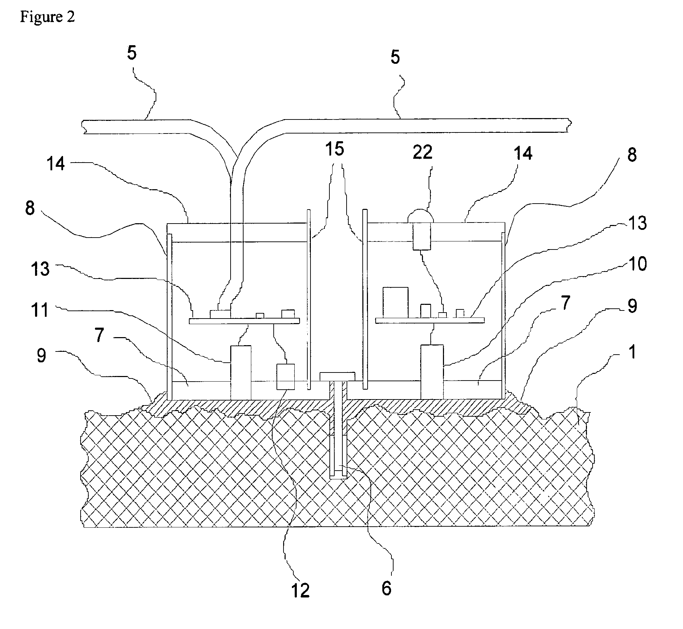

Referring to drawing 4, there is monitoring device, with substantially the same components as the embodiment described with reference to figure 2, however, with differences as follows. A radio antenna, 16, is fixed into the tope plate, 14, and connected electrically to appropriate parts of the electronic circuits, 13. There is no cable used with this embodiment. A piezoelectric bi-material device, 17, converts vibrational energy and temperature changes into electrical energy, with the vibrational energy coming from the operating noise in the structure and temperature changes coming from weather changes and the movement of the sun in the sky. In this embodiment the electro-mechanical transmitter and receivers, 10 and 11, are also used as sources of electrical energy coming from vibrational energy in the structure. Wind energy is converted into electrical energy by a small generator, 18, driven by a rotating wind-vane, 19. A solar cell, 20, is a further source of energy, in this particular embodiment implemented as a flexible panel fitted to the outside of the housing of the monitoring device. Electrical energy is stored in a re-chargeable battery, 21. To assist in conserving energy the electronic circuit, 13, switches-off as many parts of the circuit as possible for as long as possible.

In use embodiment B is used substantially as embodiment A with differences mainly due to the uncertainty of its environmental power sources. It is possible that a monitoring device will fail to operate because there is insufficient energy stored in its battery. This occurs if the schedule of tests is too frequent for the environmental power sources to sustain. Consequently, it is possible for data to be lost from the schedule of tests and therefore the decision-making algorithms and the risk analysis tree must be robust against loss of data.

### In embodiment C, in which:

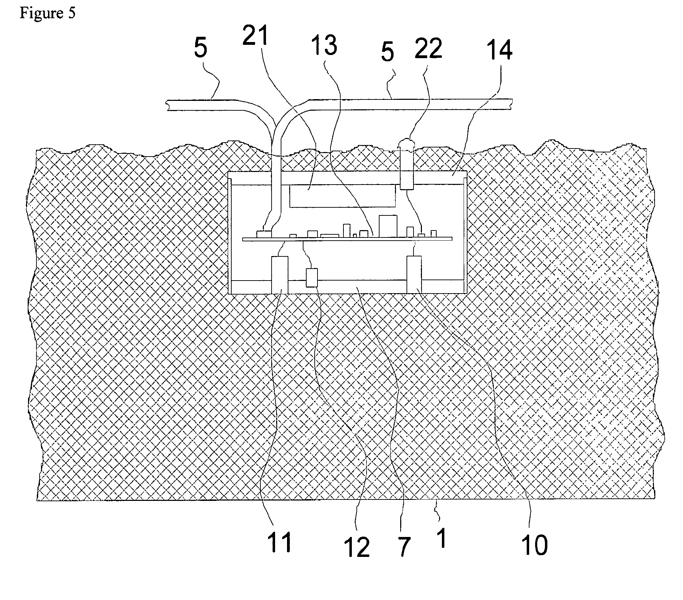

Figure 5 shows a sketch of the principal components in a monitoring device for use partially or substantially or totally embedded into a concrete structure.

Referring to drawing 5, there is a monitoring device, which is either partially or substantially or totally embedded into a concrete structure, 1, so that it needs no other means of support. The monitoring device has a mating plate, 7. It is important that the concrete covers the sound or ultrasound transducers, 10 and 11, and is mechanically joined to them to allow ultrasound to pass easily between the monitoring device and concrete structure, 1. The monitoring device can be embedded when the structure is being made, during pouring, or it can be added after construction by creating an oversize hole then pumping concrete into the hole around the monitoring device. Each monitoring device has two transducers, 10 and 11, acting as transmitter and receiver respectively. The transmitter and receiver, 10 and 11, are fixed and sealed into the mating plate, 7. A small temperature sensor, 12, is fixed in the mating plate close to the surface with the mortar. Electronic circuits, 13, are held inside the enclosure, 8. The mating plate, 7, enclosure, 8, and top plate, 14, protect the internal parts of the monitoring device against penetration of concrete and water or other environmental agents whose presence on the electronic circuits, 13, might cause them to perform not as intended. A cable, 5, brings power and communications but it is also possible not to use a cable and instead to use radio communications with an antenna (not shown in figure 5), preferably but not necessarily, protruding from the surface of the concrete, 1, then the kind of environmental power sources described in embodiment B are used, although the degree of embedding in the concrete will affect how easily a solar panel and wind vane can be mounted. A signalling lamp, 22, extending out from the top plate so that it is visible even if the monitoring device is otherwise embedded in the concrete and not visible, is used to identify the location of the monitoring device. In all other respects the embodiment is substantially the same as embodiment A.

In use embodiment C can be used substantially as embodiment A if cable power is used, otherwise embodiment C can be used with radio and environmental energy sources in which case it is used substantially like embodiment B.

### In embodiments D, in which:

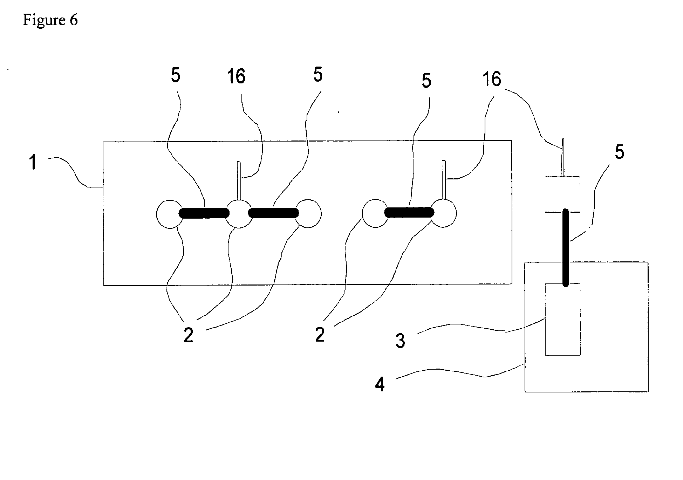

Figure 6 shows a schematic diagram of a monitoring system using both cable and radio communications, comprising several monitoring devices, 2, fixed to a structure, some monitoring devices, 2, with only cable communications, 5, and some monitoring devices, 2, having both radio, 16, and cable communications means, 5, performing a second function as a communicating device between one or a plurality of monitoring devices, 2, and an archive, 3.

### In embodiment E, in which:

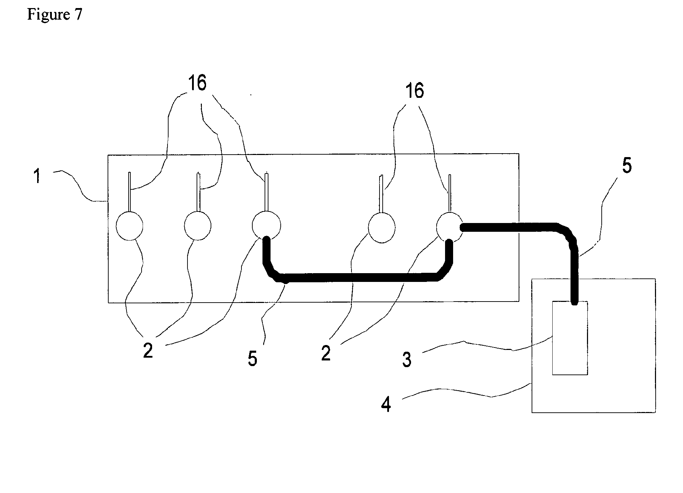

Figure 7 shows a schematic diagram of a monitoring system using both cable and radio communications, comprising several monitoring devices, 2, fixed to a structure, 1, some monitoring devices, 2, with only radio communications means, 16, and some monitoring devices having both radio and cable communications, performing a second function as a communicating device able to communicate with a single archive device, 3, by cable, 5.

Referring to figures 6 and 7, monitoring devices can be clustered and/or arranged to communicate in a variety of different ways using both radio and cables. The best configuration depends upon the structure under test and the availability of power (for cable) and the distances to be covered by a particular communication method. It is possible to make local clusters of monitoring devices and communicating devices all communicating by cable, with one or more communicating devices also using radio to communicate to an archive device, for example. It is also possible to make local clusters of monitoring devices and communicating devices all communicating by radio, with one or more communicating devices also using cable to communicate to an archive device, 3, kept in a building or shelter, 4.

In use embodiments D and E can be used substantially as described by embodiments A and/or B as appropriate to the means of communication and means of provision of electrical power.

### In embodiment F, in which:

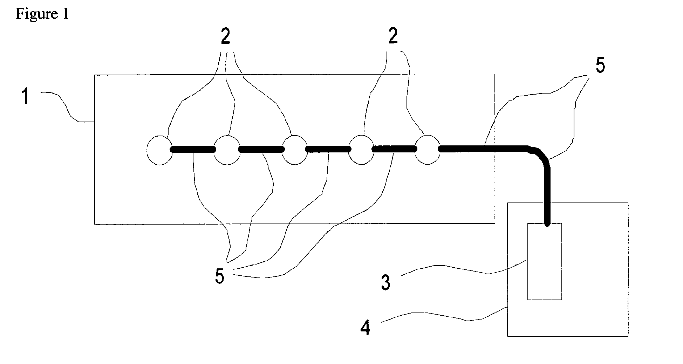

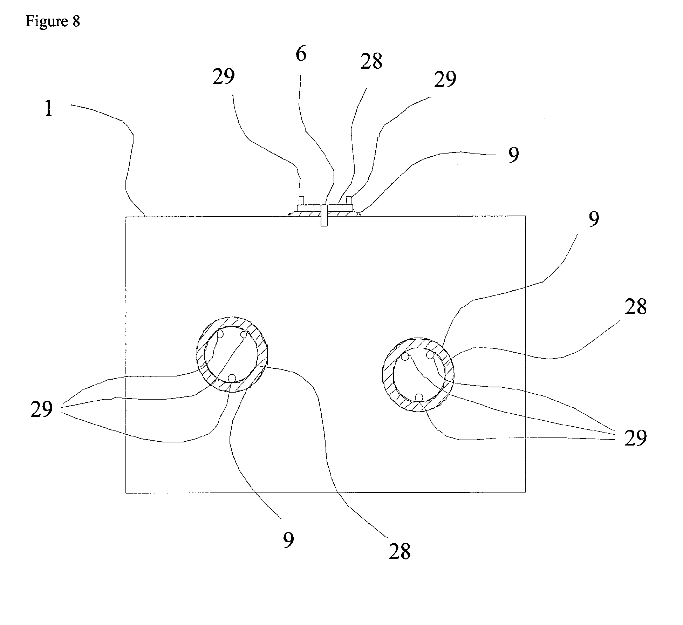

Figure 8 shows a structure surface, 1, under test with attachment devices in the form of plates, 28, fixed to the surface of the structure. For simplicity an archive device is not shown here but it is used as shown in figures 1 and 2, with the added possibility that the archive may not be live, by which is meant that it may not be able to process signals until the operator returns to it, in which case the archive could be kept distant from the structure being monitored and one archive device could be used in more than one monitoring system.

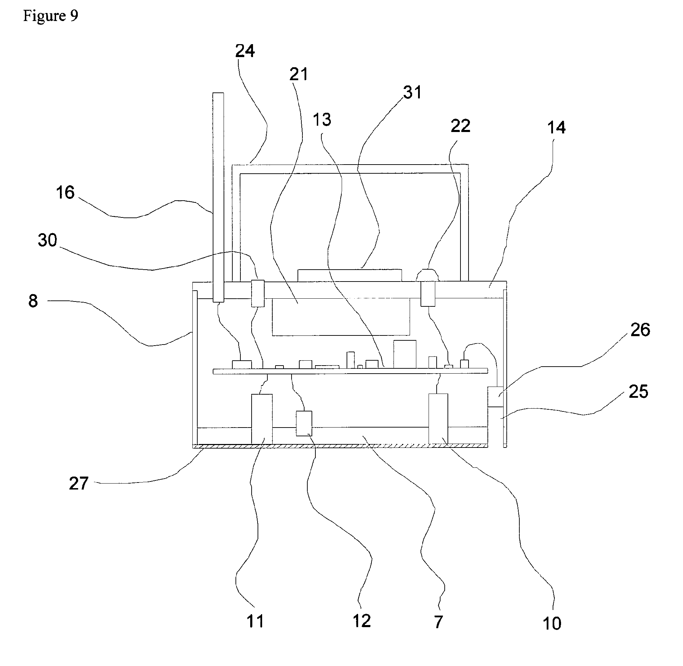

Figure 9 shows a demountable monitoring device.

Referring to figure 8, a plurality of attachment plates, 28, have been fixed to the structure, 1, at locations chosen by an expert for monitoring. An adhesive coupling agent, 9, is used to fill the space between the surface of the structure, 1, and the attachment plate, 28. The attachment plate is also held in place by an anchor bolt, 6, which alone supports the weight of the attachment plate while the adhesive coupling agent is hardening. The attachment plate, 28, also has three locating pillars, 29, arranged to fit into the demountable monitoring device in only one way so that the transmitter and receiver therein always actuate at precisely the same locations on the attachment plate, 28.

Referring to figure 9, a demountable monitoring device is substantially as described in embodiment A but with the following differences. It has a handle, 24, for an operator to hold for carrying the demountable monitoring device when it is demounted and to aid in mounting and demounting the monitoring device onto and from attachment plates, 28. It has a battery, 21, preferably but not necessarily rechargeable, to provide electrical power to the electronic circuits, 13; a socket, 30, to allow a cable to be connected for the purpose of possibly providing electrical power to recharge the battery, 21, and possibly for the purpose of communicating with an archive device, 3; an antenna, 16, to allow the monitoring device communicate with an archive device, 3, as an alternative to cable communications; a lamp, 22, to indicate the progress of testing and communications and not for the purpose of identifying the monitoring device since there is preferably only one monitoring device but a plurality of attachment plates, 28, and monitoring locations; it has a second coupling agent, 27, to assist sound waves and ultrasound waves travelling between the transmitter and receiver transducer, 10 and 11, and the attachment plate, 28; it has a keypad, 31; it has three cavities, 25, with proximity switches, 26, above each cavity, 25.

In use embodiment F is different in one important respect in the way it operates from embodiments A, B, C, D and E substantially due to the fact that, preferably but not necessarily, only one monitoring device is used as a roving device carried by an operator to test at a plurality of testing locations defined by the attachment plates, 28. The differences are therefore substantially associated with the process of testing at each test location but there is little substantial difference in the way the signals collected are processed. The operator aligns the monitoring device correctly on an attachment plate, 28, by matching-up the male pillars, 29, on the attachment plate with female cavities, 25, large enough to allow the male pillars, 29, to enter. It is preferable but not essential to have proximity switches, 26, or micro-switches or some similar means capable of signalling to the electronics, 13, when a pillar, 29, is fully home in a cavity, 25; when all three pillars, 29, are fully home then the electronic circuits detect this event and testing or monitoring at that location can begin. A second coupling agent between the attachment plate, 28, and the mating plate, 7, is preferable but may not be essential and this could be a conventional coupling get if the mating surfaces of the attachment plate, 28, and the mating plate, 7, are sufficiently smooth and parallel to allow good coupling of sound and ultrasound waves without a coupling agent. In all other respects the operation of embodiment F is substantially the same as embodiment A. The same monitoring device can be used at more than one structure if necessary. Embodiment F is generally used when low installation costs are desirable and higher annual monitoring costs can be tolerated because embodiment F requires an operator to be employed whereas the other embodiments operate substantially automatically. Roving use of a monitoring device necessitates that the frequency of monitoring at any particular location on a structure probably cannot be high, the frequency could possibly be as low as once a year, however, this may still be sufficiently frequent for some structures. Roving use of a monitoring device also necessitates that the monitoring locations, 28, the locations of the attachment plates, are readily accessible to the operator.

When frequent monitoring is needed of inaccessible monitoring locations it is preferable to use one of the other embodiments.

## Claims

1. A system for monitoring the mechanical integrity, operational worthiness and safety of a structure with: (a) at least one and preferably a plurality of monitoring devices, each with means for being attached and/or coupling to the structure to be monitored, so that a known pattern of sound waves and/or ultrasound waves can pass from one or more electro-mechanical transmitter transducers in one or possibly more monitoring devices into the structure being monitored and so that echo-waves of sound and/or ultrasound from the surface and interior of the structure being monitored can pass into one or possibly more monitoring devices to be converted there by one or possibly more electro-mechanical receiver transducers into electrical signals and then, preferably but not essentially, converted into digital signals, preferably but not essentially, signals processed to enhance the quality of the information relating to echo-waves therein, with, preferably but not essentially, means to store at least one and possibly more echo-wave signals in each monitoring device; (b) means for communicating information, including information from echo-wave signals, from one or more and preferably all the monitoring devices in a monitoring system to possibly one or more communicating devices or to possibly one or more of the monitoring devices but ultimately to preferably one but possibly more than one device to store or archive information derived from echo-wave signals from one or a plurality or preferably all the monitoring devices capable of collecting echo-wave signals on the structure to be monitored; (c) means in a monitoring system for analysing or interpreting some or all of the information pertaining to echo-waves collected by one or more and preferably all the inspection devices monitoring the structure to be monitored and preferably but not necessarily to compare the information, possibly but not essentially after some processing, with other processed or archived information in order to decide if any significant change in the mechanical integrity, operational worthiness and safety of the structure being monitored has happened; (e) means to alert an operator or a person or persons or a system about any changes in mechanical integrity, operational worthiness and safety of the structure being monitored; (f) means for performing some or preferably all the tasks of a monitoring system partially or preferably fully automatically; (g) means for providing power to the various monitoring devices, communicating devices and archive devices requiring power to function properly.

2. A system for monitoring the mechanical integrity, operational worthiness and safety of a structure, as claimed in claim 1, with monitoring devices placed at locations on the structure to be monitored that are preferably but not essentially chosen by a structural engineer or a person with expertise related to what the monitoring system is intended to monitor.

3. A system for monitoring the mechanical integrity, operational worthiness and safety of a structure, as claimed in claims 1 to 2, with means both for transmitting and for receiving sound or ultrasound waves from one or a plurality of monitoring devices; it is also possible but not necessary for one electro-mechanical transducer in any monitoring device to be used both for transmitting and for receiving sound or ultrasound waves.

4. A system for monitoring the mechanical integrity, operational worthiness and safety of a structure, as claimed in claims 1 to 3, with a monitoring device or monitoring devices preferably but not essentially attached to the surface of the structure being monitored for a period of time that is equal to or greater than the desired monitoring period.

5. A system for monitoring the mechanical integrity, operational worthiness and safety of a structure, as claimed in claims 1 to 4, with possibly one or possibly more demountable monitoring devices, which can be moved between test locations.

6. A system for monitoring the mechanical integrity, operational worthiness and safety of a structure, as claimed in claims 1 to 5, having one or possibly more monitoring devices, possibly but not necessarily demountable monitoring devices, preferably but not essentially used with one or a plurality of permanently fixed attachment devices on the structure being monitored to aid or simplify the mounting or demounting, of an inspection device thereupon, possibly but not necessarily with the attachment device being a simple, low-cost attachment, for example a plate, fixed to the structure to mark or locate or otherwise define the precise location of transmitting and/or receiving sound waves or ultrasound waves, with the attachment preferably but not necessarily assisting in simplifying the coupling of sound waves or ultrasound waves into or out of the structure.

7. A system for monitoring the mechanical integrity, operational worthiness and safety of a structure, as claimed in claims 1 to 6, having one or a plurality of monitoring devices each with a mating surface part, preferably but not necessarily a plate, which is used next to the surface of the structure being monitored.

8. A system for monitoring the mechanical integrity, operational worthiness and safety of a structure, as claimed in claims 1 to 7, with any gap between the surface of a structure being monitored and the mating surface of a monitoring device or the mating surface of an attachment device filled with a coupling agent to aid the efficient transmission of sound or ultrasound between the monitoring device or the attachment device and the structure being monitored, preferably but not necessarily with the mating surface made of a material to which the coupling agent adheres; preferably but not necessarily with the coupling agent made from a material that also adheres to the material of which the structure, under the mating device, is made, with the coupling agent preferably but not essentially filling the space between the sound or ultrasound transmitter and/or receiver in the monitoring device and the structure surface.

9. A system for monitoring the mechanical integrity, operational worthiness and safety of a structure, as claimed in claims 1 to 8, with a coupling agent that hardens to fix and hold one or a plurality of monitoring devices or the mating surfaces of one or a plurality of attachment devices in place on the surface of the structure under test for a relatively long period of time, preferably but not essentially for as long as the period of monitoring, so that the coupling agent supports the weights of any attached devices and withstands any operational or other forces that might be exerted on the same devices.

10. A system for monitoring the mechanical integrity, operational worthiness and safety of a structure, as claimed in claims 1 to 9 with a hardened coupling agent that is, preferably but not essentially, acoustically matched to the material of which the structure being monitored is made under the mating surface of any monitoring devices or attachment devices.

11. A system for monitoring the mechanical integrity, operational worthiness and safety of a structure, as claimed in claims 1 to 10, having one or a plurality of monitoring devices or attachment devices with preferably but not essentially two or possibly more means to support the weights of any monitoring devices or attachment devices that are fixed to a structure being monitored.

12. A system for monitoring the mechanical integrity, operational worthiness and safety of a structure, as claimed in claims 1 to 11, having one or a plurality of monitoring devices or attachment devices with one means or preferably more of supporting the weight of any monitoring device or attachment device, of which one supporting means in particular functions quickly to support the weight and any operational or other forces that might be exerted on any monitoring device.

13. A system for monitoring the mechanical integrity, operational worthiness and safety of a structure, as claimed in claims 1 to 12, intended for monitoring a structure made in part or entirely of concrete, preferably but not essentially with those parts of a monitoring device, which carry or convey sound waves or ultrasound waves, made in part or entirely out of a composite material of a hardened mixture of epoxy resin or a similar thermo-setting or thermo-plastic polymer and alumina powder or some similar mineral powder, which preferably but not essentially has the properties of: bonding to mortar and many other adhesives and being acoustically matched to concrete.

14. A system for monitoring the mechanical integrity, operational worthiness and safety of a structure, as claimed in claims 1 to 13, intended for monitoring a structure made in part or entirely of concrete, with preferably but not essentially mortar or similar adhesives as a coupling agent, preferably but not essentially with the abilities: to adhere to concrete, to be acoustically matched to concrete and to flow viscously when first prepared, closing any gaps between transducers, mating plates and attachment devices and any uneven surface of the concrete at the test location but without substantially flowing away from therein.

15. A system for monitoring the mechanical integrity, operational worthiness and safety of a structure, as claimed in claims 1 to 14, having one or a plurality of monitoring devices or one or a plurality of attachment devices with preferably but not essentially one hole or possibly more holes in each mating plate thereof, possibly but not necessarily located in the centre of the mating plate, preferably but not essentially with means of rapidly holding any monitoring device or any attachment device onto the surface of the structure being monitored using the hole or holes to support the weights of the monitoring devices or attachment devices.

16. A system for monitoring the mechanical integrity, operational worthiness and safety of a structure, as claimed in claims 1 to 15, having one or a plurality of monitoring devices or one or a plurality of attachment devices, each with preferably but not essentially one or a plurality of anchor bolts or similar fixing means to hold any monitoring devices or any attachment devices in place on the surface of the structure being monitored.

17. A system for monitoring the mechanical integrity, operational worthiness and safety of a structure, as claimed in claims 1 to 16, having one or a plurality of monitoring devices, each with an enclosure to protect the electronic circuits of each monitoring device.

18. A system for monitoring the mechanical integrity, operational worthiness and safety of a structure, as claimed in claims 1 to 17, having one or a plurality of monitoring devices, each with an enclosure to protect the electronic circuits therein that is impervious to water, other chemicals, dust, the rays of the sun, wind and that it should be rugged to withstand a variety of environments, preferably but not essentially for many years.

19. A system for monitoring the mechanical integrity, operational worthiness and safety of a structure, as claimed in claims 1 to 18, with cable, preferably an electrical cable or possibly a fibre-optic cable, for conveying information and possibly power between one or more and preferably all monitoring devices in the monitoring system and possibly to other communicating devices and preferably to one or possibly more archive devices and possibly to one or more sources of electrical power. It is further possible to share one or possibly more cables between two or a plurality or preferably all monitoring devices, archives and communicating devices in such a pattern as to reduce or substantially to minimize the total length of cable used in a monitoring system; one preferred arrangement of cable involves linking each monitoring device to a nearby neighbouring monitoring device, preferably but not essentially connecting other communicating devices in the same way and preferably but not necessarily with at least one archive device connected in the same way.

20. A system for monitoring the mechanical integrity, operational worthiness and safety of a structure, as claimed in claims 1 to 19, with cable for conveying information and possibly power, preferably an electrical cable or possibly a fibre-optic cable, with clusters of monitoring devices connected by cable to a communicating device, with the communicating device linked possibly to other communicating devices but ultimately to at least one or possibly more archive devices and possibly to one or more sources of electrical power.

21. A system for monitoring the mechanical integrity, operational worthiness and safety of a structure, as claimed in claims 1 to 20, with radio communication means.

22. A system for monitoring the mechanical integrity, operational worthiness and safety of a structure, as claimed in claims 1 to 21, with substantially the same message-handling protocol for communicating between monitoring devices, communication devices and archive devices, which protocol, preferably but not essentially, seeks to avoid collisions between messages and is, preferably but not essentially, able to recover from messages corrupted by collisions between messages from different devices or other causes of interference.

23. A system for monitoring the mechanical integrity, operational worthiness and safety of a structure, as claimed in claims 1 to 22, with one or a plurality of internal batteries in one or a plurality of monitoring devices or communication devices, preferably but not essentially, when a monitoring device or a communication device does not have a cable to provide electrical power thereto.

24. A system for monitoring the mechanical integrity, operational worthiness and safety of a structure, as claimed in claims 1 to 23, with one or a plurality of internal, re-chargeable batteries in one or a plurality of monitoring devices or communication devices and means to re-charge the batteries.

25. A system for monitoring the mechanical integrity, operational worthiness and safety of a structure, as claimed in claims 1 to 24, with one or a plurality of means to generate power from the environment possibly for inspection devices and possibly for communication devices, including: possibly means to convert power from the rays of the sun into electrical energy; means to convert power from the wind into electrical power; possibly means to convert vibrational energy in the structure into electrical power, using possibly but not necessarily one or more piezoelectric or electro-magnetic devices; possibly means to convert thermal gradients or temperature changes in the vicinity of the monitoring device into electrical power, using one or more Peltier effect devices, or pyro-electric devices, or layered material devices, with dissimilar thermal expansion coefficients in the materials and at least one layer being a piezoelectric device.

26. A system for monitoring the mechanical integrity, operational worthiness and safety of a structure, as claimed in claims 1 to 25, with one or possibly a plurality of known patterns of sound waves or ultrasound waves transmitted into the structure being monitored, using electro-mechanical transducers in each of those monitoring devices with the capability to transmit sound waves or ultrasound waves.

27. A system for monitoring the mechanical integrity, operational worthiness and safety of a structure, as claimed in claims 1 to 26, with means to detect preferentially one or possibly a plurality of known patterns in any sound or ultrasound echo-waves received from the structure being monitored, as collected by electro-mechanical receiver transducers in each of those monitoring devices with the capability to receive, resulting from sound or ultrasound waves transmitted into the structure under test by an inspection device with the capability to transmit.

28. A system for monitoring the mechanical integrity, operational worthiness and safety of a structure, as claimed in claims 1 to 27, it is preferable but not essential to transmit one or a plurality of semi-continuous sine-waves each of constant amplitude and frequency as patterns of sound waves or ultrasound waves from one or a plurality of inspection devices.

29. A system for monitoring the mechanical integrity, operational worthiness and safety of a structure, as claimed in claims 1 to 28, it is preferable but not essential to detect any patterns of semi-continuous sine-waves, each of constant amplitude and frequency, in an echo-wave signal collected by one or a plurality of receiver transducers in one or a plurality of inspection devices, each using a narrow, band-pass filter having a centre frequency equal to the known transmitted frequency, preferably but not essentially, using digital frequency synthesis of the transmitted sine-wave and, preferably but not essentially, using a lock-in amplifier method capable of tracking any changes in the frequency of the transmitted sine-wave, preferably but not essentially measuring the absolute amplitude of the received sine-wave signal and preferably but not necessarily measuring the relative phase angle of the received sine-wave signal compared with the transmitted wave, preferably but not essentially testing at a plurality of frequencies and grouping the results so generated into one set for subsequent processing and archiving.

30. A system for monitoring the mechanical integrity, operational worthiness and safety of a structure, as claimed in claims 1 to 29, it is preferable but not essential to transmit from one or a plurality of inspection devices one or a plurality of patterns of known bursts of waves of finite duration, known herein and more widely as chirps, with one or a plurality of receiver transducers synchronized to start collecting chirp echo-wave signals with some controlled delays, preferably but not necessarily zero time delay after each chirp is first transmitted, with each chirp covering, preferably but not essentially, as wide a range of frequencies as possible but limited to the range of frequencies within which the transmitter and receiver transducers in any monitoring device or devices have useful sensitivity and further limited to the useful range of frequencies that can be transmitted through the material of which the structure being monitored is made.

31. A system for monitoring the mechanical integrity, operational worthiness and safety of a structure, as claimed in claims 1 to 30, with means to detect preferentially patterns of a finite duration burst of waves or chirps in an echo-wave signal from one or a plurality of inspection devices, resulting in short, simple event pulses for the arrival of each chirp echo-wave, with wider bandwidth chirps generally but not necessarily resulting in shorter event pulses and better resolution of echoes and hence reflectors in the structure being monitored, making it simpler for the monitoring system to identify structural changes in the structure being monitored.

32. A system for monitoring the mechanical integrity, operational worthiness and safety of a structure, as claimed in claims 1 to 31, particularly but not necessarily structures made partly or entirely of concrete, transmitting preferred chirp patterns of sound waves or ultrasound waves from one or a plurality of inspection devices with each chirp having a swept-frequency lasting a time, preferably but not essentially, one or more periods of the waves transmitted and, preferably but not essentially, in the range 30 microseconds to 300 microseconds and covering a range of frequencies somewhere in the range from 10 kHz to 500 kHz.

33. A system for monitoring the mechanical integrity, operational worthiness and safety of a structure, as claimed in claims 1 to 32, with means to process echo-waves collected by receiver transducers in one or a plurality of inspection devices in several stages when chirp patterns are being transmitted, the first two preferred stages of processing being, after, possibly, signal amplification and filtering using analogue electronic circuits: averaging and pattern detection, with these stages, preferably but not necessarily, being done in digital signal representation, it being relatively unimportant in which order averaging and pattern recognition are performed provided the pattern recognition process is a linear operation because then both averaging and pattern recognition are linear operations and commute with the operation of addition but it being important in which order averaging and pattern recognition are performed if the pattern recognition process is non-linear, when averaging should, preferably but not necessarily, be performed first.

34. A system for monitoring the mechanical integrity, operational worthiness and safety of a structure, as claimed in claims 1 to 33, with a preferred method of pattern recognition for use with chirp echo-wave signals from inspection devices sometimes called matched filtering, being the optimum linear signal processing method for detecting a known burst of waves, in which matched filtering can make use of either: (a) a copy of the electrical excitation pattern to the transmitter, or (b) preferably but not essentially, a part of the signal collected during a sound or ultrasound calibrating test on a simple homogeneous sample, having the same or similar acoustic properties to the structural material of interest, with the matching chirp as accurately matched as possible to the chirp pattern found in echo-wave chirp signals from the structure under test.

35. A system for monitoring the mechanical integrity, operational worthiness and safety of a structure, as claimed in claims 1 to 34, with matched filtering means for detecting chirps in echo-wave signals resulting from spike excitation applied to transmitters in any monitoring devices, in which the matched filter is based upon a pattern of sound or ultrasound waves recorded in a calibration experiment on a simple homogeneous sample, having the same or similar acoustic properties to the structural material of interest.

36. A system for monitoring the mechanical integrity, operational worthiness and safety of a structure, as claimed in claims 1 to 35, with preferably but not essentially, means to perform a stage of processing to follow averaging and pattern recognition to convert bipolar signals derived from echo-waves from the structure being monitored into unipolar signals, a preferred method is to calculate the magnitude of the analytic function of the signal but other possible methods include simple rectification or taking the magnitude of the signal, these latter two methods are preferably but not essentially followed by low-pass filtering or band-pass filtering to smooth the signal.

37. A system for monitoring the mechanical integrity, operational worthiness and safety of a structure, as claimed in claims 1 to 36, having one or possibly a plurality of monitoring devices with each having, preferably but not essentially, a data processing device which is either: a computer or a microprocessor or a digital signal processor or a microcontroller or combinations of the aforementioned devices or substantially similar devices along with suitable memory and other electrical circuits, with the data processing device preferably but not necessarily controlling some or possibly all of the following functions: the transmission of any sound and ultrasonic waves, the receiving of any sound or ultrasound echo-waves from the structure being monitored, the setting of analogue gain levels in amplifiers, the conversion of any received signals into digital information signals, processing of signals, storing of signals, communicating information ultimately to an archive device.

38. A system for monitoring the mechanical integrity, operational worthiness and safety of a structure, as claimed in claims 1 to 37, having one or possibly more archive devices with each archive device having one or possibly a plurality of data processing devices, which is either: a computer or a microprocessor or a digital signal processor or a microcontroller or some similar device of combinations of the aforementioned devices, with the data processing device having means to perform preferably but not necessarily all of the following functions: communication with monitoring devices and communication devices, archiving of information from monitoring devices, signal processing, decision-making about whether or not any significant structural change has occurred based upon information or signals from one or a plurality of inspection devices and archive information or signals, combining of results of two or more decisions relating to different monitoring locations on the structure being monitored thereby creating a decision for a larger part or parts of the structure or of the whole structure, communicating results of decision-making to an operator or to one or more persons or to a system responsible for safety or repair of the structure.

39. A system for monitoring the mechanical integrity, operational worthiness and safety of a structure, as claimed in claims 1 to 38, with means to collect and archive signals from one or a plurality of monitoring devices on the structure being monitored at a time when the structure is known to be free of significant structural faults or when the structure is known to contain some known faults, which are in some sense acceptable, with signals collected, preferably but not necessarily, when the monitoring system is first installed, preferably but not necessarily, after or before independent tests verify that the structure is free of structural faults or contains known faults that are in some sense acceptable.

40. A system for monitoring the mechanical integrity, operational worthiness and safety of a structure, as claimed in claims 1 to 39, with means for decision-making, preferably but not necessarily, using algorithmic means known as an artificial neural network, in which case one artificial neural network is specially trained to make decisions about one specific monitoring location, with one or a plurality of artificial neural networks used, with the basis of the decision-making being to compare a signal from a particular monitoring location, preferably but not necessarily processed to reduce interference, operational noise and to enhance the resolution of sound or ultrasound echoes from structural components in the structure being monitored, with two or more sets of grouped signals derived from the same monitoring location in substantially the same format, with two of the aforementioned sets being in particular derived from: (a) experimental, preferably processed, signals to reduce operational noise and to enhance the quality of echoes, collected before significant mechanical change and/or signals collected with known mechanical change, (b) preferably some or possibly all of the same signals as in (a) but importantly all from the same monitoring location as in (a) and importantly all made to be representative of significant mechanical change, or migrated as referred to herein, with the decision-making algorithm deciding which set the signal under consideration for decision-making is most like, preferably but not essentially, with the result of the decision given in the form of a probability that the signal under consideration belongs to the set **characterized by** significant structural change.

41. A system for monitoring the mechanical integrity, operational worthiness and safety of a structure, as claimed in claims 1 to 40, with means to process copies of archive signals from monitoring devices taken when the structure is in a known condition, possibly but not necessarily a state free of faults or a state with known acceptable faults and to modify them, by a process referred to as migration herein, so that migrated signals becomes representative of the structure when in a mechanical or structural state that is significantly changed, with means to use migrated signals, preferably but not necessarily, for training or conditioning or in some way adapting a decision-making algorithm used by the monitoring system to classify signals.

42. A system for monitoring the mechanical integrity, operational worthiness and safety of a structure, as claimed in claims 1 to 41, with means to process or migrate signals by: (a) possibly adding or possibly multiplying or possibly adding and multiplying or possibly multiplying and adding a plurality of mean RMS levels of white-noise or pink-noise to the signal being migrated, (b) determining the ordinate positions of any peaks in the information signal and causing the positions to be moved by a random amount in possibly either one or possibly both ordinates.

43. A system for monitoring the mechanical integrity, operational worthiness and safety of a structure, as claimed in claims 1 to 42, with means to process or migrate signals by a process called FEM migration herein, which is **characterized by** the capability for an expert to create migrated signals to reflect precise structural changes in the structure being monitored, with a preferable but not essential method of FEM migration being substantially as follows: (a) a finite element modelling (FEM) program is used to create a geometrically accurate, local model of the part of the structure being monitored in contact with a specific inspection device of interest, a dimensional accuracy of +/-2% can be achieved generally in the model, (b) the FEM program is then run to predict the electrical signal to be received by the receiver transducer in the inspection device under consideration in response to the excitation applied to the transmitter signal, using the geometry model and the material properties of the various materials locally in the structure and preferably the material properties of transmitter and receiver transducers, (c) it is generally true that the material properties typically required by FEM for each different material used in the model are density (+/-10%), Young's modulus (+/-25%), shear modulus (+/-50%) and acoustic or ultrasonic attenuation (+10000% to - 99%), shown in parentheses are the typical errors for these values that might be known at the start of the modelling process, the larger errors in material properties are likely to cause a greater difference between a migrated signal and the unmigrated experimental signal than errors in dimensions, preferably but not necessarily, with means of a separate controlling program, the FEM program is run repeatedly with a fixed geometry model but with material properties varied by the controlling program and chosen to vary within an error range specified by the expert so that a plurality of provisionally migrated signals may be created, (d) after a newly migrated provisional signal is generated by the FEM program it is preferable but not essential to compare it with the unmigrated experimental signal so that a scalar error value is calculated which quantifies any differences, (e) the controlling program has means to try to minimize this error by choosing new values of material properties so that subsequent provisionally migrated signals have lower errors, (f) the controlling program continues varying the material properties until an acceptably low error has been achieved, at which point and the provisionally migrated signal should appear to be substantially like the unmigrated experimental signal, it is probable that the material properties used in the last FEM model are more accurate representations of the values for the material properties of the part of the structure under consideration, so that error has been reduced substantially, (g) the expert introduces one or a plurality of different changes into the FEM model for the part of the structure under consideration, possibly but not necessarily, representing faults which it would be desirable for the monitoring system to detect, and the FEM program is run once for each change, resulting in one or preferably a plurality of new migrated signals for that test location, (h) the process can be repeated for, preferably but not necessarily, more or all of the monitoring locations in a monitoring system.

44. A system for monitoring the mechanical integrity, operational worthiness and safety of a structure, as claimed in claims 1 to 43, having a controlling program used for FEM migration with means or methods for minimizing preferably a scalar value but possibly a vector value that is dependent upon several variables including, preferably but not necessarily, means or methods known as: a genetic algorithm, a self-annealing algorithm or any other minimization means or methods used for minimizing scalar values dependent upon several variables.

45. A system for monitoring the mechanical integrity, operational worthiness and safety of a structure, as claimed in claims 1 to 44, with means to combine two or more risks, preferably but not necessarily in the form of probabilities of significant structural change as reported by decision-making means using signals derived from monitoring devices at specific locations on a structure being monitored, so that the combined risk gives a risk of significant structural change for larger parts of the structure or possibly for the entire structure being monitored and for risks to be combined in a way that is controlled by an expert on the ultimate purpose or use or application of the monitoring system so as to yield a result which is of importance for that purpose and which requires the combined risk and structural change to be reported to an operator, or persons, or other system for possible action to be taken.

46. A system for monitoring the mechanical integrity, operational worthiness and safety of a structure, as claimed in claims 1 to 45, with means to display a computer-generated image of the structure being monitored, preferably but not necessarily, with representation of three dimensions, showing the positions on the representation of the structure of at least one and preferably but not necessarily all the monitoring devices, with representations of the monitoring devices and means to indicate the classification of the structure for each monitoring device so represented, possibly but not necessarily, using false colours such as green for no structural change and red for structural change, so that an operator can quickly assess the location of any structural change.

47. A system for monitoring the mechanical integrity, operational worthiness and safety of a structure, as claimed in claims 1 to 46, having one or a plurality of monitoring devices with means for measuring the temperature of the surface of the structure being monitored and for this temperature value to be included with or attached to received echo-wave signals, ultimately to form a part of the information to be submitted to decision-making means for the purpose of training or conditioning the decision-making means or for the purpose of making a decision using a trained or pre-conditioned decision-making means.

48. A system for monitoring the mechanical integrity, operational worthiness and safety of a structure, as claimed in claims 1 to 47, having one or possibly a plurality of monitoring devices with means for signalling their presence amongst two or a plurality of monitoring devices, for the purpose of identifying the location on the structure being monitored of any particular monitoring device, with the means for signalling being, preferably but not necessarily, optical means or possibly audible means or possibly electromagnetic means or possibly mechanical means or any other kind of identifiable means.

49. A system for monitoring the mechanical integrity, operational worthiness and safety of a structure, as claimed in claims 1 to 48, having a monitoring device with means of signalling its presence that is a lamp visible from the outside of the monitoring device, which can be flashed at a speed under the control of electronic circuits within the monitoring device and, preferably but not necessarily, with means for the flashing to be modulated to contain information, preferably but not necessarily with the modulation representing an association with the identifier or name or address or some such similar identifier of the monitoring device whose presence is being identified.

50. A system for monitoring the mechanical integrity, operational worthiness and safety of a structure, as claimed in claims 1 to 49, using cable communications with different possible means for connecting possibly but not necessarily monitoring devices, possibly but not necessarily communicating devices and possibly but not necessarily archive devices to the cable, including: (a) two electrical sockets in the housing or enclosure of a device so that suitable cables can be simply plugged into each socket, (b) a short length of cable emerging from the housing or enclosure of a device with the free end of the short cable being electrically connected to a longer cable travelling between other devices forming the monitoring system.

51. A system for monitoring the mechanical integrity, operational worthiness and safety of a structure, as claimed in claims 1 to 50, particularly structures made partly or entirely of concrete, with one or a plurality of inspection devices embedded in the concrete of which the structure is made, preferably but not essentially, close to an outer surface of the structure through which communications means are possible, possibly in the form of communications using a cable or possibly using communications by radio or some other electro-magnetic wave, with means to provide power to each inspection device possibly through a cable or possibly derived from environmental sources.

52. A system for monitoring the mechanical integrity, operational worthiness and safety of a structure, as claimed in claims 1 to 51, and substantially as described hereinbefore, especially with reference to one or more of the figures shown in the accompanying drawings.
